**Europäisches Patentamt**

**European Patent Office**

**Office européen des brevets**

(19)

(11) Veröffentlichungsnummer: **0 319 593**
**A1**

# (12) EUROPÄISCHE PATENTANMELDUNG

Veröffentlicht nach Art. 158 Abs. 3 EPÜ

(21) Anmeldenummer: **88906228.7**

(22) Anmeldetag: **23.06.88**

Daten der zugrundeliegenden internationalen Anmeldung:

(86) Internationale Anmeldenummer:
**PCT/SU88/00136**

(87) Internationale Veröffentlichungsnummer:
**WO88/10104 (29.12.88 88/28)**

(51) Int. Cl.³: **A 61 F 2/24**

(30) Priorität: **25.06.87 SU 4261094**

(43) Veröffentlichungstag der Anmeldung:
**14.06.89 Patentblatt 89/24**

(84) Benannte Vertragsstaaten:
**BE DE GB IT LU NL SE**

(71) Anmelder: **BUKATOV, Alexandr Semenovich**
**pl. Pobedy, 1-A-122**
**Moscow, 121293(SU)**

(71) Anmelder: **AGAFONOV, Andrei Vasilievich**
**ul. Smolnaya 63-149**
**Moscow, 125445(SU)**

(71) Anmelder: **KOSTRETSOV, Anatoly Stepanovich**
**ul. Bakuninskaya, 10/12-7**
**Moscow, 107005(SU)**

(71) Anmelder: **DZEVISHEK, Elena Viktorovna**
**ul. Musy Dzhalilya, 29/1-111**
**Moscow, 115580(SU)**

(71) Anmelder: **IOFIS, Naum Abramovich**
**Lomonosovsky pr. 23-416**
**Moscow, 117311(SU)**

(71) Anmelder: **SHUMAKOV, Valery Ivanovich**
**ul. Smolenskaya 7-56**
**Moscow, 119121(SU)**

(71) Anmelder: **KAIDASH, Arnold Nikolaivich**
**ul. Tashkentskaya, 21-1-66**
**Moscow, 109472(SU)**

(71) Anmelder: **POPOV, Leonid Leonidovich**
**ul. Pervomaiskaya 21-34**
**Moskovskaya obl. Dolgoprudny, 141700(SU)**

(71) Anmelder: **KOZYRKIN, Boris Ivanovich**
**Zelenograd, 913-124**
**Moscow, 103575(SU)**

(54) **HERZKLAPPENPROTHESE.**

(57) Die Herzklappenprothese enthält einen Klappenring (1) mit einer Öffnung (2) zum Blutdurchfluß und eine in dieser Innenöffnung eingesetzte Scheibenklappe (3), die zwischen Abstützungen (4, 5) zum schließen und Öffnen der Klappenöffnung (2) schwimmend eingesetzt ist. Die Scheibenklappe (3) ist mit einem asymmetrischen, veränderlichen Profil ausgeführt, die beispielsweise einen konvexen Flächenabschnitt (9) und konkaven Flächenabschnitt (10) besitzt, durch welche Abschnitte mit dem Klappenring (1) zusammen Zonen eines unterschiedlichen hydraulischen Widerstandes für den Blutdurchfluß entstehen und demzufolge auch eine Winkelverschwenkung der Scheibenklappe (3) zustandekommt, wodurch die Lebensdauer der Herzklappenprothese verlängert werden kann.

FIG.1

(71) Anmelder: JURECHKO, Vladimir Nikolaevich
ul. Botanicheskaya, 33/8-106
Moscow, 127276(SU)

(71) Anmelder: ZHAROV, Sergei Vasilievich
Sovetsky pr. 52-21
Karaganda, 470023(SU)

(71) Anmelder: KOROTEEVA, Irina Viktorovna
Frunzenskaya nab. 50-223
Moscow, 119270(SU)

(71) Anmelder: CHARKOVSKY, Alexandr Vladimirovich
Frunze pr. 32-41
Vitebsk, 210023(SU)

(72) Erfinder: BUKATOV, Alexandr Semenovich
pl. Pobedy, 1-A-122
Moscow, 121293(SU)

(72) Erfinder: AGAFONOV, Andrei Vasilievich
ul. Smolnaya 63-149
Moscow, 125445(SU)

(72) Erfinder: KOSTRETSOV, Anatoly Stepanovich
ul. Bakuninskaya, 10/12-7
Moscow, 107005(SU)

(72) Erfinder: DZEVISHEK, Elena Viktorovna
ul. Musy Dzhalilya, 29/1-111
Moscow, 115580(SU)

(72) Erfinder: IOFIS, Naum Abramovich
Lomonosovsky pr. 23-416
Moscow, 117311(SU)

(72) Erfinder: SHUMAKOV, Valery Ivanovich
ul. Smolenskaya 7-56
Moscow, 119121(SU)

(72) Erfinder: KAIDASH, Arnold Nikolaivich
ul. Tashkentskaya, 21-1-66
Moscow, 109472(SU)

(72) Erfinder: POPOV, Leonid Leonidovich
ul. Pervomaiskaya 21-34
Moskovskaya obl. Dolgoprudny, 141700(SU)

(72) Erfinder: KOZYRKIN, Boris Ivanovich
Zelenograd, 913-124
Moscow, 103575(SU)

(72) Erfinder: JURECHKO, Vladimir Nikolaevich
ul. Botanicheskaya, 33/8-106
Moscow, 127276(SU)

(72) Erfinder: ZHAROV, Sergei Vasilievich
Sovetsky pr. 52-21
Karaganda, 470023(SU)

(72) Erfinder: KOROTEEVA, Irina Viktorovna
Frunzenskaya nab. 50-223
Moscow, 119270(SU)

(72) Erfinder: CHARKOVSKY, Alexandr Vladimirovich
Frunze pr. 32-41
Vitebsk, 210023(SU)

(74) Vertreter: Nix, Frank Arnold, Dr.
Kröckelbergstrasse 15
D-6200 Wiesbaden(DE)

HERZKLAPPENPROTHESE

## Technisches Gebiet

Die vorliegende Erfindung bezieht sich auf die Medizin, insbesondere auf Kardiochirurgie, und zwar auf eine Herzklappenprothese, die zum Einsetzen anstelle einer beschädigten natürlichen Herzklappe bestimmt ist.

## Zugrundeliegender Stand der Technik

In den letzten Jahren finden sogenannte Scheibenprothesen der Herzklappe immer mehr eine weitgehende Anwendung, die einen Klappenring bzw. - körper mit einer Öffnung zum Blutdurchfluß einschließen, in der eine Scheibenklappe eingesetzt wird, die zum Zumachen und Öffnen der Innenöffnung im Arbeitsablauf der Herzklappe vorgesehen ist. Die erwähnte Scheibenklappe wird vermittels einer oberen Stütze und unteren Abstützungsanordnung bzw. der sogenannten Hubbegrenzer schwimmend angeordnet. Die Hubbegrenzer treten mit einer oberen bzw. Distalseite und einer unteren bzw. Proximalseite der Scheibenklappe in Berührung, von denen die Distalseite eine konvexe ist und eine Vertiefung zur Aufnahme des oberen Hubbegrenzers aufweist, während die andere, die Proximalseite in Übereinstimmung mit der Konfiguration des unteren Hubbegrenzers ausgeführt ist, wie sie beispielsweise die Patentschrift der USA Nr. 4 057 857, herausgegeben für Fettel et al., oder Patentschrift der USA Nr. 4 713 071, herausgegeben für Iofis et al. wiedergeben.

Derartige Herzklappenprothesen weisen jeweilige Vorteile auf, die darin bestehen, daß eine oberflächliche Berührung der Scheibenklappe in deren sowohl öffnender als auch zuschließender Stellung mit den Hubbegrenzern erreicht wird. Jedoch tritt die Scheibenklappe bei dem Arbeitsablauf mit den Hubbegrenzern an ein und denselben Flächenabschnitten in Berührung, was eine Ab-

Kürzung ihrer Lebensdauer und demzufolge auch der Lebensdauer der ganzen Herzklappe verursacht, da die Arbeitsdauer der Herzklappenprothese in erster Linie mit der Lebensdauer der Scheibenklappe zusammenhängt. Aus diesem Grunde in der US-PS Nr. 4 713 071 wird die Benutzung einer oberen Begrenzerstütze mit schräger Fläche vorgesehen, die unter einem Winkel zwischen $1^O$ bis $3^O$ zu der der Längsebene der erwähnten Begrenzerstütze senkrechten Ebene verlegt wird, was von der Strömungsdynamik des Blutstromes ausgehend eine nur geringe Winkelverschwenkung der Scheibenklappe in Bezug auf ihre Eigenachse bewirken soll. Obwohl die erwähnte Ausführung auch eine Steigerung der Lebensdauer der Scheibenklappe der Herzklappenprothese durch die periodische Abwechselung der Berührungsflächenabschnitte an der Scheibenklappe und der Hubbegrenzer während des Arbeitsablaufs erreichen läßt, ist sie mit gewissen Nachteilen behaftet. Einer von diesen besteht darin, daß durch den erwähnten Bereich der Schrägstellungen der Ebenen nur eine verhältnismäßig geringe Winkelverschwenkung der Scheibenklappe erreicht wird, während eine Schrägstellungszunahme durch die aufbaumäßige Bedingungen unmöglich ist, obwohl in mehreren Fällen eine Vergrößerung des Ausschlages der Winkelverschwenkung bzw. Drehung vom Standpunkt der Lebensdauer sowie aus hämodynamischen Gründen wünschenswert ist. Darüber hinaus befindet sich die Schrägfläche in einer schwerzugänglichen Stelle, während nach deren Fertigstellung ein zusätzliches hochqualitatives Polieren der erwähnten Ebene durchgeführt werden muß, was wesentliche Komplikationen in die verfahrenstechnischen Herstellungsvorgänge unter Vergrößerung des Arbeitsaufwandes der Herstellung der ganzen Herzklappenprothese und demzufolge auch unter Steigerung deren Kosten mit sich bringt.

Offenbarung der Erfindung

Der Erfindung liegt die Aufgabe zugrunde, die Scheibenklappe in der Herzklappenprothese derart auszuführen, daß diese Scheibenklappe selbsttätig eine Eigenwinkelverschwenkung mit vergrößerter Amplitude bewirkt und in ihrer Herstellung fertigungsgerecht ist.

Die erwähnte technische Aufgabe wird dadurch gelöst, daß in der Herzklappenprothese, die einen Klappenring mit einer Öffnung für den Blutdurchfluß und eine in dieser Innenöffnung eingesetzte Scheibenklappe einschließt, die eine Distal- und Proximalseite aufweist welche beiden mit den Hubbegrenzern in Zusammenwirkung treten, erfindungsgemäß die Distalseite mit einem asymmetrischen veränderlichen Profil versehen ist, dessen verschiedene Abschnitte mit dem Klappenring zusammen Zonen unterschiedlichen hydraulischen Widerstandes gegenüber dem Blutdurchfluß entwickeln.

Beim Vorhandensein von Zonen unterschiedlichen hydraulischen Widerstandes des Blutdurchflußes entsteht ein Drehmoment, durch welches die Scheibenklappe um die Eigenachse herum nur infolge deren eigener aufbaumäßiger Gestaltung gedreht oder unter einem Winkel verschwenkt wird, ohne daß die anderen Bauteile der Herzklappenprothese einer Bearbeitung bedürfen, insbesondere ohne Ausarbeitung einer Schrägfläche an dem oberen Hubbegrenzer, wie sie US-PS Nr. 4 713 071 vorsieht, welche Bauteile sich an schwerzugänglichen Stellen befinden.

Dadurch ermöglicht die erfindungsgemäße Ausführung eine Vereinfachung des Herstellungsverfahrens der ganzen Herzklappenprothese. Da das veränderliche Profil an der Scheibenklappe ausgeführt wird, die als ein Einzelteil gesondert gefertigt wird, ist es selbstverständlich, daß an deren Distalseite Profile selbst unterschiedlichster Konfiguration leicht ausgeführt werden können, indem somit ein weiter Bereich der Verschwenkungswinkel der Scheibenklappe erreicht wird, während bei der Benutzung der Schrägfläche nach der US-PS Nr. 4 713 071 diese Winkelverschwenkung durch die erwähnte Schrägfläche eingeschränkt ist. Die erwähnte profilierte Oberfläche kann in denselben Ausrüstungen gefertigt werden, die zur Herstellung einer gewöhnlichen Scheibenklappe der bereits bekannten Herzklappenprothese dienen . Die erfindungsgemäße Scheibenklappe kann auch bei den bekannten Ausführungen der Scheibenherzklappenprothesen unter Erzielung hoher Zuverlässigkeit und langer Lebensdauer eingesetzt werden, ohne zusätzlicher Ausrüstungen zu bedürfen.

Bei der einfachsten Ausführungsversion ist an einem Teil der Distalseite eine konvexe Fläche wie eine Sphäre vorgesehen, während an dem Restteil der Distalseite eine konkave Fläche ausgeführt wird. Diese Ausführungsvariante stellt eine im verfahrenstechnischen Sinne einfachste Variante dar und ermöglicht außerdem noch, das Eigengewicht der Scheibenklappe der Herzklappenprothese ein wenig herabzusetzen, was ja insbesondere bei einer starken Schwächung der Tätigkeit der Muskelschicht des Herzens von besonderer Wichtigkeit ist.

Zur Entwicklung der besten Verhältnisse für die Winkelverschwenkung der Scheibenklappe können über die Kreislänge der Scheibenklappe beabstandet angeordnete Radialrillen ausgenutzt werden. Dabei kann eine der Flanken der Radialrillen in der durch die Symmetrieachse der Scheibenklappe verlaufenden Diametralebene gelegt werden, während die andere Flanke zu dieser schräggestellt werden kann. Die Anzahl der erwähnten Radialrillen bestimmt die gewünschte Winkelverschwenkung der Scheibenklappe und kann eine gerade bzw. ungerade Zahl erreichen. Jedoch liegen die Schrägflanken der zueinander diametralliegenden Radialrillen bei gerade Anzahl der Radialrillen auf entgegengesetzten Seiten der durch die in dieser Diametralebene befindlichen normalen Flanken der betreffenden Radialrillen gehenden Diametralebene. Zum Vermeiden der Thrombenbildung sind die Radialrillen im Querschnitt mit abgerundeten Rändern und Rippen zweckmäßigerweise zu versehen. Mit demselben Zweck ist eine parabolische Form in Radialrichtung dem Rillenboden zu verleihen, welche die Entfernung des in die Radialrille geratenen Blutes begünstigt.

Gemäß einer anderen der Ausführungsvarianten wird die Ausbildung der Distalseite als eine Kombination von krummlinigen konvexen und in diese übergehenden flachen Abschnitten vorgesehen, wobei die erwähnten flachen Abschnitte unsymmetrisch verteilt werden. Eine solche Ausführung ist am zweckmäßigsten für die Fertigung. Am zweckmäßigsten ist eine derartige Gestalt der Distalseite bei Benutzung der Herzklappenprothese anzufertigen, deren Scheibenklappe eine sphärische Distaloberfläche besitzt, während die flachen Abschnitte durch Wegfräsen von Abflachungen auf der betreffenden sphärischen Oberfläche herzustellen sind.

So können auch vorhandene Scheibenklappen ohne wesentliche Zusatzaufwendungen unter einer wesentlichen Steigerung deren Zuverlässigkeit vervollkommnet werden.

Die Distalseite der Scheibenklappe kann auch wie ein unregelmäßiger Pyramidenstumpf derart ausgeführt werden, daß dessen Spitze bei dem Einsetzen der Scheibenklappe im Klappenring in Bezug auf die Achse des genannten Klappenringes versetzt wird.

In einen weiteren Ausführungsvariante wird die Anfertigung einer Spiralrille vorgesehen, die von der Mitte der Scheibenklappe aus zu deren Umfangskreis verläuft, wobei diese Form die Winkelverschwenkung nur durch eine Spiralrille ermöglicht. Bei der Benutzung einer solchen Spiralrille in der Herzklappenprothese, deren Scheibenklappe an der Distalseite eine Mittelvertiefung zur Aufnahme des oberen Hubbegrenzers besitzt, kommuniziert diese Spiralrille an ihrem der Scheibenmitte zugewandten Ende mit der erwähnten Vertiefung zwecks Einführung des oberen Hubbegrenzers beim Zusammenbau der Herzklappenprothese in die betreffende Rille an dieser Einmündung, während an ihrem anderen Ende die Spiralrille offen bleibt, wodurch der Zusammenbauvorgang wesentlich vereinfacht wird.

### Kurzbeschreibung der Zeichnungen

Nachstehend wird die Erfindung anhand der Beschreibung konkreter, die vorliegende Erfindung aber nicht einschränkender Ausführungsvarianten der Erfindung und der angelegten Zeichnungen näher erläutert; in diesen zeigt:

Fig. 1 erfindungsgemäße Herzklappenprothese, deren Scheibenklappe einen konvexen und einen konkaven Abschnitt aufweist, im Schnitt;

Fig. 2 bedingte Verteilung der Größe des Stirnwiderstandes in Abhängigkeit vom Profil der Scheibenklappe, die Fig. 1 wiedergibt;

Fig. 3 und 4 erfindungsgemäße Scheibenklappe der Herzklappenprothese in schematischer, deren grundsätzliche Ausführung erklärender Darstellung;

Fig. 5 Scheibenklappe gemäß einer anderen erfindungsgemäßen Ausführungsvariante, in der an ihrer Distalseite Radialrillen ausgespart sind;

Fig. 6 Draufsicht der erfindungsgemäßen Herzklappenprothese aus der Fig. 5;

Fig. 7 das Profil der Radialrille veranschaulichender Schnitt längs Linie VII-VII aus der Fig. 6;

Fig. 8 Herzklappenprothese gemäß einer weiteren Ausführungsvariante der Erfindung im Schnitt schematisch dargestellt, bei der an der sphärischen Distalseite der Scheibenklappe Abflachungen bzw. Facetten ausgeführt sind;

Fig. 9 Draufsicht der Scheibenklappe aus der Fig. 8;

Fig. 10 noch eine erfindungsgemäße der möglichen Ausführungsvarianten der Herzklappenprothese;

Fig. 11 Draufsicht der erfindungsgemäßen Herzklappenprothese mit Spiralrille an der Distalseite der Scheibenklappe; in schematischer Darstellung;

Fig.12 Schnitt XII-XII aus der Fig. 11.

### Beste Ausführungsvariante der Erfindung

In der nachstehenden Beschreibung sind die gleichen Aufbauteile der Herzklappenprothese mit ein und denselben Bezugsziffern bezeichnet, während die Bauteile gleicher Zweckbestimmung, die sich aber in ihrer aufbaumäßigen Ausführung unterscheiden, eine zusätzliche Bezeichnung mit Buchstaben aufweisen.

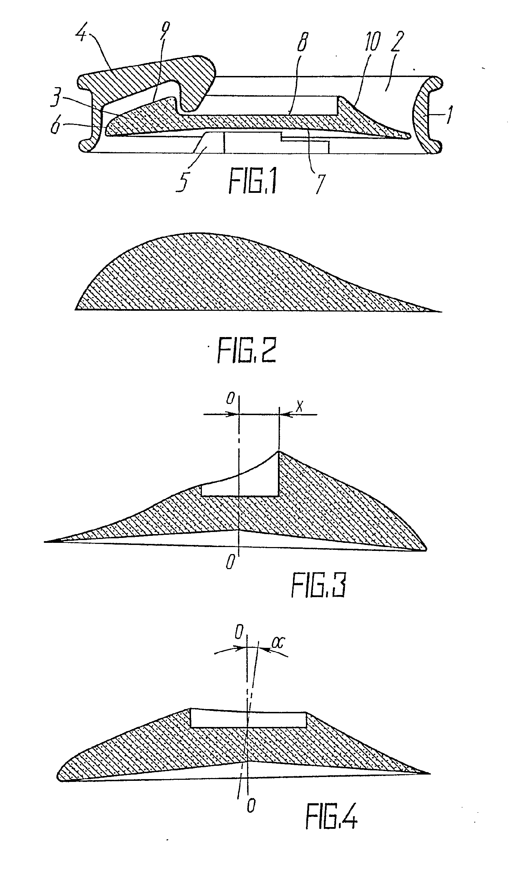

Zunächst betrachten wir Fig. 1, in der die Ausführung der Herzklappenprothese im Ganzen gemäß einer der Ausführungsvarianten der Erfindung gut zu ersehen ist. Wie aus dieser Fig. 1 zu ersehen ist, enthält die Herzklappenprothese einen Klappenring bzw. -körper 1, der in Form eines Zylinders mit Bünden ausgeführt ist, die zur Befestigung einer aus den Zeichnungen nicht ersichtlichen, um deren Überlastung durch überflüssige, in keiner Beziehung mit der Erfindung stehende Einzelheiten zu vermeiden, Manschette vorgesehen sind. Die Ausführung, Gestaltung und der Stoff einer solchen Manschette sind für die in dem betreffenden Gebiet erfahrenen Fachleute gut bekannt. Diese Manschette dient der Befestigung der Herzklappenprothese selbst an den Umgebungsgeweben bei dem Einsetzen der Prothese. Der Klappenring umgibt die Klappeninnenöffnung 2 und bildet noch den Sitz für die Scheibenklappe 3. Die betreffende Scheibenklappe 3 wird schwimmend eingesetzt, ähnlich wie sie in der in der vorliegenden Beschreibung als Druckschriftenhinweis erwähnten US-PS Nr. 4 713 071 ausführlich beschrieben ist, und in diesem Zustand vermittels der Hubbegrenzer in Form von der oberen Stütze 4 und unteren Abstützungsanordnung 5 zurückgehalten.

Die Scheibenklappe 3 weist eine obere bzw. Distalseite 6 und eine untere bzw. Proximalseite 7 auf. Die Proximalseite 7 ist leicht konkav ausgeführt, während deren Krümmungsradius in Übereinstimmung mit der Konfiguration der unteren Abstützungsanordnung 5 gewählt ist. In dem Mittelteil der Distalseite 6 ist eine Vertiefung 8 zur Aufnahme der oberen Stütze 4 ausgespart.

Wie die Fig. 1 deutlich wiedergibt, ist der auf der Zeichnung linke Teil 9 der Scheibenklappe 3 konvex und in derselben Zeichnung rechte Teil 10 der Scheibenklappe 3 konkav ausgeführt, wodurch die Distalseite 6 der Scheibenklappe 3 ein veränderliches Profil aufweist, dessen unterschiedliche Teile bzw. Flächenabschnitte 9 und 10 mit dem Klappenring zusammen Zonen eines veränderlichen hydraulischen Widerstandes für den durch den Klappenring hindurchströmenden Blutdurchfluß bilden. In der Fig. 2 ist die Verteilung des erwähnten hydraulischen Widerstandes des Blutdurchflusses über den Querschnitt der Scheibenklappe 3 in schematischer Darstellung und vergrößertem Maßstab wiedergegeben. Unter der Wirkung des erwähnten veränderlichen hydraulischen Widerstandes des Blutdurchflusses entsteht ein Drehmoment, durch welches die Winkelverschwenkung der Scheibenklappe 3 um deren Eigenachse herum, welche im Raum zwischen der oberen Stütze 4 und unteren Abstützungsanordnung 5 frei schwimmt, geschieht.

Die Herstellung der Scheibenklappe 3 mit dem genannten Querschnittsprofil kann vermittels der mechanischen Bearbeitung des Ausgangswerkstücks in zwei Etappen durchgeführt werden. Während der ersten Etappe wird ein halbsphärischer Rohling und während der zweiten Etappe an dem gewählten Flächenabschnitt die Konkavität fertiggestellt, wobei diese konkaven Abschnitte durch entweder eine lineare Verstellung des Bearbeitungswerkzeugs bezüglich der Grundachse 0-0 des Werkstücks auf die Größe "X", die im allgemeinen zwischen 30 $\mu$m und 700 $\mu$m liegt, wie sie die Fig. 3 wiedergibt, oder Winkelverstellung des Bearbeitungswerkzeugs in Bezug auf die Grundachse 0-0 um den Winkel "$\alpha$" gefertigt werden, der im allgemeinen zwischen 30' bis 3° beträgt, wie es in der Fig. 4 dargestellt ist.

Die genannten asymmetrischen Abschnitte des konvexen und konkaven Profils können sich gegeneinander flächengemäß unterschiedlich verhalten, je nach der erforderlichen Winkelverschwenkung der Scheibenklappe 3.

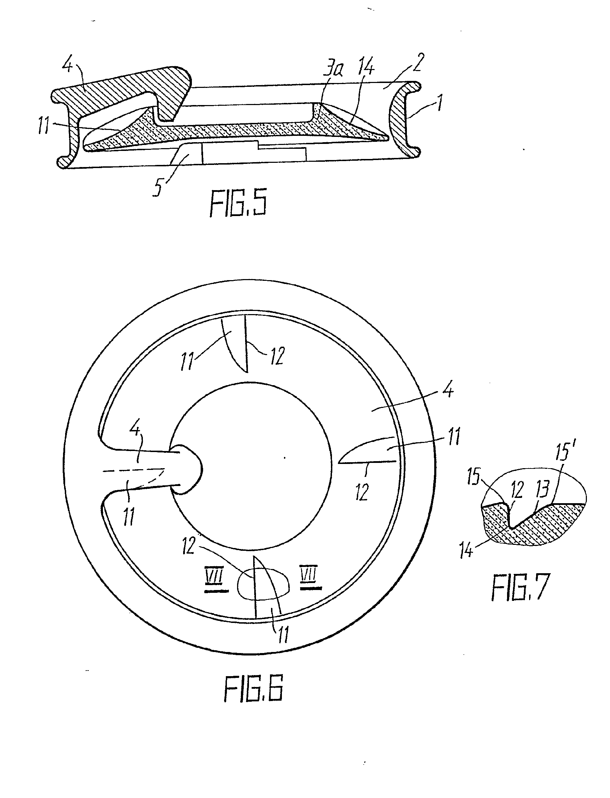

In Fig. 5 und 6 ist eine andere Ausführungsvariante der Herzklappenprothese im Schnitt und in Draufsicht dargestellt, die ebenso wie die Klappenprothese aus der Fig. 1 einen Klappenring 1 mit der Klappenöffnung 2, eine obere Stütze 4 und untere Abstützungsanordnung 5 einschließt, zwischen denen die Scheibenklappe 3a eingesetzt ist. In dieser Scheibenklappe 3a sind Ausnehmungen bzw. Radialrillen 11 ausgespart. Jede solche Radialrille 11 besteht aus einer in der durch die Eigensymmetrieachse der Scheibenklappe 3a hindurchgehenden Diametralebene liegenden Normalflanke 12 (Fig. 7) und einer zu der Normalflanke unter einem Winkel verlegten Schrägflanke 13, die mit der Normalflanke 12 am Schnittpunkt den Rillenboden 14 bildet. Wie sie in der Fig. 7 dargestellt sind, werden die Ränder 15 und 15' sowie der Boden 14 der Radialrille 11 abgerundet, während in Radialrichtung der Rillenboden 14 eine parabolische Gestaltung aufweist, wie ihn die Fig. 5 gut wiedergibt.

Die in der Fig. 6 dargestellte Scheibenklappe 3a besitzt vier zueinander diametralverteilte Radialrillen 11. In diesem Fall liegen die Schrägflanken 13 der diametralliegenden Radialrillen 11 auf entgegengesetzten Seiten der durch die Symmetrieachse der Scheibenklappe 3a hindurchgehenden Diametralebene. Durch die betreffende aufbaumäßige Gestaltung der Distalseite der Scheibenklappe 3a entsteht ein veränderliches Profil, dessen verschiedene Abschnitte, und zwar die konvexen Abschnitte und die Radialrillen 11 mit dem Klappenring zusammen Zonen eines unterschiedlichen Widerstandes des Blutdurchflusses bilden, durch welche ein die Verdrehung bzw. Winkelverschwenkung der Scheibenklappe 3a bewirkendes Drehmoment entsteht.

Obwohl die in Fig. 5 und 6 dargestellte Ausführungsvariante die Ausfräsung nur vier Radialrillen 11 vorsieht, kann deren Anzahl mehr oder weniger Radialrillen tatsächlich erreichen. Jedenfalls müssen bei Benutzung von Radialrillen in gerader Anzahl kommen die Schrägflanken der diametralliegenden Radialrillen 11 auf entgegengesetzten Seiten der durch die Eigensymmetrieachse der Scheibenklappe 3a gehenden Diametralebene liegen.

Selbstverständlich kann auch die Scheibenklappe mit einer ungeraden Anzahl der Radialrillen verwendet werden, die über die Länge der Scheibenkreise gleichmäßig verteilt sind, obwohl diese Variante aus den Zeichnungen nicht ersichtlich ist, und zwar kann eine Scheibenklappe mit drei gegeneinender unter dem Winkel 120° verteilten Radialrillen ausgenutzt werden.

Es muß darauf aufmerksam gemacht werden, daß die Anzahl der Radialrillen mit der notwendigen Schnelligkeit der Winkeldrehung der Scheibenklappe zusammenhängt, die der funktionelle Zustand der Muskelschicht des Herzens bestimmt. Für eine geschwächte Muskelschicht des Herzens muß die Winkeldrehung der Scheibenklappe kleiner sein. Die Belastungsgröße der Muskelschicht des Herzens kann durch Veränderung des Schrägstellungswinkels der Schrägflanke 13 gegen die Normalflanke 12 eingestellt werden, wobei je größer dieser Winkel wird, desto kleiner wird die Belastung. Es sei betont, daß auch die parabolische Gestaltung des Rillenbodens in Radialrichtung nicht unbedingt ausgeführt zu werden braucht, sondern sie ist nur eine bevorzugte Gestalt, da sie eine Herabsetzung der Turbulenz während des Arbeitsablaufs der Herzklappenprothese bewirkt.

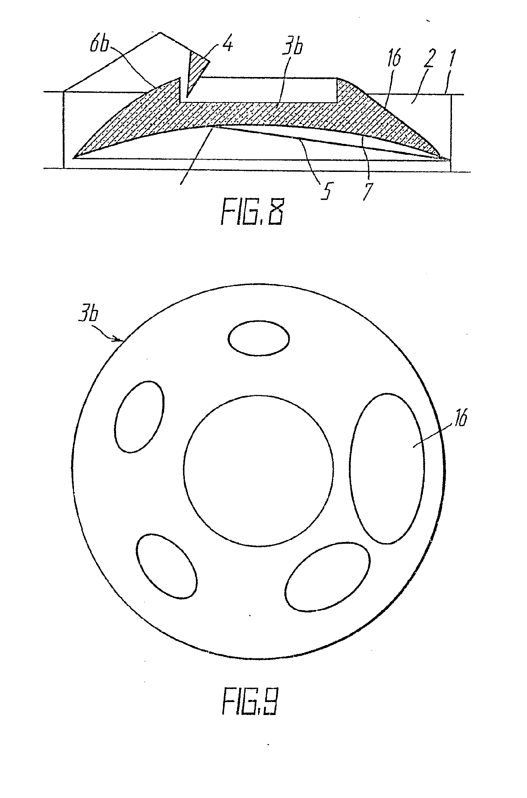

Die in der Fig. 8 und Fig. 9 dargestellte Herzklappenprothese unterscheidet sich von den obenbeschriebenen nur durch Ausführung der Scheibenklappe 3b.

Die Distalseite dieser Scheibenklappe 3b ist sphärisch ausgeführt und an dieser sind flachen, Facetten 16 bildende Abflachungen weggefräst, wie sie aus der Fig. 9 und im Schnitt aus der Fig. 8 (nur eine Facette hier) gut zu ersehen sind. Die erwähnten Facetten 16 können unterschiedlich gross sein und ungleichmäßig verteilt werden, wie sie die Fig. 9 wiedergibt. Bei einer gleichmäßigen Verteilung der an der Oberfläche der Distalseite der Scheibenklappe 3b ausgefrästen Facetten 16, was durchaus möglich ist, muß die Gesamtanzahl der Radialrillen dabei bevorzugt eine ungerade Zahl erreichen. Im allgemeinen muß die Bedingung erfüllt werden, daß im Querschnitt die Scheibenklappe 3b an ihrer Distalseite keinesfalls eine achssymmetrische Gestaltung aufweist. Nur in diesem Fall durch das veränderliche Profil der Distalseite der Scheibenklappe mit Hilfe des Klappenrings zusammen entstehen die Zonen des unterschiedlichen hydraulichen Widerstandes für den Blutdurchfluß, was wiederum die Winkelverschwenkung der Scheibenklappe verursacht. Es ist darauf zu achten, , daß zwecks Vermeidung einer Verwirbelung des Blutstromes die obenerwähnten flachen Facetten 16 an der sphärischen Oberfläche knickfrei in diese übergehen müssen. Technologisch gesehen hängt die Erfüllung der Anforderungen an die Qualität der Oberfläche der Distalseite der Scheibenklappe 3b mit keinen Schwierigkeiten zusammen.

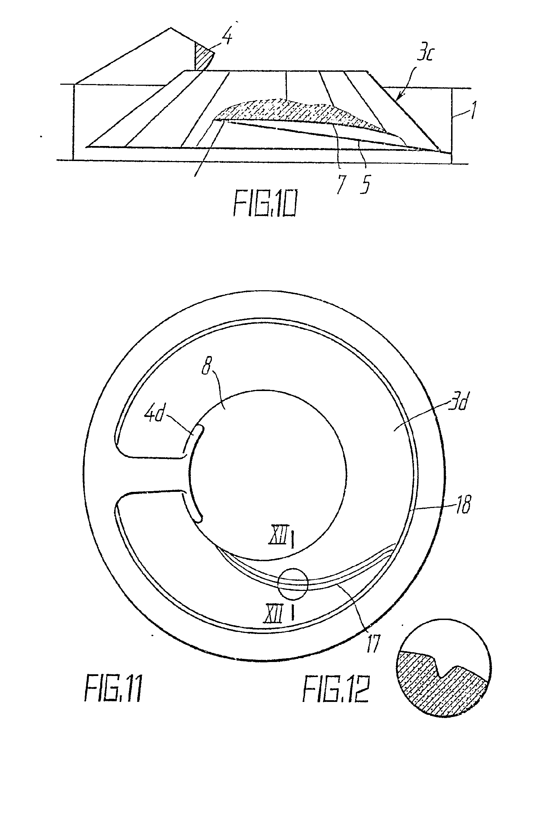

In der durch die Fig. 9 dargestellten Ausführungsvariante sind die flachen Facetten hintereinander mit den krummlinigen Abschnitten abwechselnd verteilt. Jedoch ist auch eine andere Ausführung der Scheibenklappe der Herzklappenprothese möglich. Diese wird in Fig. 10 gezeigt, in der die Distaloberfläche der Scheibenklappe 3c einen schrägen Pyramidenstumpf darstellt, dessen Spitze von der Achse des Klappenringes abweicht. Bei einer solchen Ausführung der Distaloberfläche der Scheibenklappe 3c besitzt sie im Querschnitt ein veränderliches Profil, durch welches die Zonen des veränderlichen hydraulischen Widerstandes für den Blutdurchfluß und demzufolge auch die Winkelverschwenkung der Scheibenklappe 3c um ihre Eigenachse herum während des Arbeitsablaufs der Herzklappenprothese zustandekommen.

In der Ausführungsversion der Scheibenklappe der Herzklappenprothese, wie sie in Fig. 11 dargestellt ist, ist die Distalseite der Scheibenklappe 3d mit einer Spiralrille 17 versehen, die von der Mittelvertiefung 8 zur Aufnahme des oberen Hubbegrenzers 4d aus bis auf die Seitenfläche der Scheibenklappe 3d verlegt ist. Diese Spiralrille 17 bewirkt die Bildung eines veränderlichen, unsymmetrischen Profils der Distalseite der Scheibenklappe 3d und demzufolge auch deren Winkelverschwenkung während des Arbeitsablaufs. Das Querschnittsprofil dieser Spiralrille ist in Fig. 12 dargestellt, aus der zu ersehen ist, daß diese abgerundete Ränder und Rillenboden aufweisen. Die betreffende Spiralrille kann auch zur Einführung der oberen Stütze 4d in die Mittelvertiefung 8 ausgenutzt werden, wenn sie an der Seite dieser Mittelvertiefung 8 und am Umfangskreis 18 offen ist, während zur Verhinderung des Herausfallens dieser oberen Stütze 4d nach ihrer Einführung durch die Spiralrille 17 aus derselben diese Spiralrille 17 längs einer Tangente an die Seitenfläche der Mittelvertiefung 8 verlegt wird. Eine solche Ausführungsversion der Scheibenklappe 3d ist am zweckmäßigsten bei der Anfertigung des Klappenkörpers und der Scheibenklappe aus Stoffen wie Keramik bzw. Einkristalle.

## Industrielle Anwendbarkeit

Die vorliegende Erfindung kann zum Ersetzen von Aorten- bzw. Mitralklappe mit bestem Erfolg ausgenutzt werden, besonders wenn die Tätigkeit des Herzens und dessen

- 13 -

Muskelschicht stark geschwächt ist. Die erfindungsgemäße Herzklappenprothese kann aus biologisch inerten
Stoffen wie beispielsweise Titanlegierungen oder Kohlenstoffmaterialien hergestellt werden.

PATENTANSPRÜCHE

1. Herzklappenprothese, die einen Klappenring (1) mit einer Öffnung (2) zum Blutdurchfluß und in dieser Öffnung schwimmend eingesetzte Scheibenklappe (3) vorsieht, die eine Distalseite (6) und Proximalseite (7) aufweist, welche beiden mit den Hubbegrenzern (4, 5) der Scheibenklappe (3) in Zusammenwirkung treten, d a d u r c h   g e k e n n z e i c h n e t, daß die Distalseite (6) ein asymmetrisches veränderliches Profil aufweist, dessen verschiedenartige Abschnitte mit dem Klappenring zusammen Zonen eines unterschiedlichen hydraulischen Widerstandes für den Blutdurchfluß bilden.

2. Herzklappenprothese nach Anspruch 1, d a d u r c h   g e k e n n z e i c h n e t, daß die Distalseite (6) der Scheibenklappe (3) einen konvexen Flächenabschnitt (9) und einen krummlinigen konkaven Flächenabschnitt (10) besitzt.

3. Herzklappenprothese nach Anspruch 1, d a d u r c h   g e k e n n z e i c h n e t, daß die Distalseite der Scheibenklappe (3a) mehrere voneinander über die Länge des Scheibenkreises beabstandete Radialrillen (11) aufweist.

4. Herzklappenprothese nach Anspruch 3, d a d u r c h   g e k e n n z e i c h n e t, daß die Scheibenklappe (3a) eine ungerade Anzahl von an der Distalseite über die Länge des Kreises der Scheibenklappe (3a) gleichmäßig verteilten Radialrillen aufweist.

5. Herzklappenprothese nach Anspruch 3, d a d u r c h   g e k e n n z e i c h n e t, daß die Scheibenklappe (3a) eine gerade Anzahl von an der Distalseite (6) ausgefrästen und gleichmäßig verteilten, diametralliegenden Rillen aufweist.

6. Herzklappenprothese nach Anspruch 3, d a d u r c h   g e k e n n z e i c h n e t, daß jede Rille eine in der durch die Eigensymmetrieachse der Scheibenklappe (3a) hindurchgehenden Diametralebene liegende Normalflanke (12) und eine unter einem Winkel zu dieser verlegte Schrägflanke (13) besitzt.

7. Herzklappenprothese nach Anspruch 5 und 6, d a - d u r c h  g e k e n n z e i c h n e t, daß die Schräg- flanken (13) der diametralliegenden Rillen auf entgegengesetzten Seiten der Diametralebene liegen, die durch durch die Normalflanken (12) dieser Rillen (11) hindurchgeht.

8. Herzklappenprothese nach Anspruch 3, d a d u r c h g e k e n n z e i c h n e t, daß die Rillen in ihrem Querschnitt keilförmig ausgeführt sind und abgerundete Ränder (15 und 15´) und Rillenboden (14) aufweisen.

9. Herzklappenprothese nach Anspruch 3, d a d u r c h g e k e n n z e i c h n e t, daß der Rillenboden (14) in Radialrichtung eine parabolische Ausführung hat.

10. Herzklappenprothese nach Anspruch 1, d a d u r c h g e k e n n z e i c h n e t, daß die Distalseite eine Kombination  konvexer krummliniger und flacher Abschnitte (16) darstellt, die mit diesen krummlinigen Flächenabschnitten vereinigt sind.

11. Herzklappenprothese nach Anspruch 10, d a - d u r c h  g e k e n n z e i c h n e t, daß die Distal- seite der Scheibenklappe (3c) eine sphärische Oberfläche mit flachen Facetten (16) darstellt.

12. Herzklappenprothese nach Anspruch 1, d a d u r c h g e k e n n z e i c h n e t, daß die Distalseite der Scheibenklappe (3c) als ein schrägstehender Pyramidenstumpf ausgeführt ist, dessen Spitze gegen die Achse des Klappenringes (2) versetzt ist.

13. Herzklappenprothese nach Anspruch 1, d a d u r c h g e k e n n z e i c h n e t, daß an der Distalseite (6d) der Scheibenklappe (3d) eine Spiralrille ausgefräst ist, die sich von der Mitte der Scheibenklappe (3d) aus bis auf den Umfangskreis (18) erstreckt.

14. Herzklappenprothese für eine Scheibenklappe mit einer Mittelvertiefung zur Aufnahme des oberen Hubbegrenzers nach Anspruch 13, d a d u r c h   g e k e n n -
z e i c h n e t, daß die          Spiralrille (17)
an ihrer dem Umfangskreis (18) der Scheibenklappe (3d)
zugewandten Seite offen ist und mit der Mittelvertiefung (8) zur Aufnahme des oberen Hubbegerenzers (4d) zu
deren Einführen in diese bei dem Zusammenbau der Herzklappenprothese kommuniziert.

1/4

FIG.1

FIG.2

FIG.3

FIG.4

FIG.5

FIG.6

FIG.7

0319593

FIG.8

FIG.9

FIG.10

FIG.11

FIG.12

# INTERNATIONAL SEARCH REPORT

International Application No PCT/SU 88/00136

**I. CLASSIFICATION OF SUBJECT MATTER** (if several classification symbols apply, indicate all) 6

According to International Patent Classification (IPC) or to both National Classification and IPC

$IPC^4$    A 61 F 2/24

**II. FIELDS SEARCHED**

| Minimum Documentation Searched 7 | |
|---|---|
| Classification System | Classification Symbols |
| $IPC^4$ | A 61 F 2/24 |

| Documentation Searched other than Minimum Documentation to the Extent that such Documents are Included in the Fields Searched 8 |
|---|
| |

**III. DOCUMENTS CONSIDERED TO BE RELEVANT 9**

| Category * | Citation of Document, 11 with indication, where appropriate, of the relevant passages 12 | Relevant to Claim No. 13 |
|---|---|---|
| A,P | US, A, 4713071, (Naum A. Iofis et al.) 15 December 1987 (15.12.87), see the claims, figures 1,2,5 cited in the description | 1 |
| A | DE, B2, 2640246, (Shiley Inc. Irvine), 25 June 1981 (25.06.81), see the claims figures 2-4,6,7 & US, A, 4057857 | 1,2,12 |
| A | US, A, 4416029, (Robert L. Kaster) 22 November 1983 (22.11.83) see the claims, figures 1,5,7-9 cited in the description | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 31 August 1988 (31.08.88) | 5 October 1988 (05.10.88) |
| International Searching Authority | Signature of Authorized Officer |
| ISA/SU | |

Form PCT/ISA/210 (second sheet) (January 1985)